(19) Europäisches Patentamt
European Patent Office
Office européen des brevets

(11) **EP 4 530 627 A1**

(12) **EUROPEAN PATENT APPLICATION**
published in accordance with Art. 153(4) EPC

(43) Date of publication:
**02.04.2025 Bulletin 2025/14**

(21) Application number: **22943638.1**

(22) Date of filing: **23.05.2022**

(51) International Patent Classification (IPC):
***G01N 33/48*** (2006.01)

(52) Cooperative Patent Classification (CPC):
**G01N 33/48**

(86) International application number:
**PCT/JP2022/021048**

(87) International publication number:
**WO 2023/228229 (30.11.2023 Gazette 2023/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **NEC Corporation**
**108-8001 Tokyo (JP)**

(72) Inventor: **OMI, Yasuo**
**Tokyo 108-8001 (JP)**

(74) Representative: **Betten & Resch**
**Patent- und Rechtsanwälte PartGmbB**
**Maximiliansplatz 14**
**80333 München (DE)**

(54) **IMAGE PROCESSING DEVICE, IMAGE PROCESSING METHOD, AND PROGRAM**

(57) In order to improve accuracy in benignancy and malignancy classification on a specimen cell in pathological diagnosis, an image processing apparatus (1) includes: a first acquisition section (11) for acquiring a target image which includes a specimen cell as a subject; a second acquisition section (12) for acquiring property information which indicates a property of a training image that has been used to train a prediction model to predict, upon receipt of input of an image including a cell as a subject, whether the cell is a benign cell or a malignant cell; and a generation section (13) for generating an image by changing a property of the target image with reference to the property information.

FIG. 1

## Description

Technical Field

The present invention relates to an image processing apparatus, an image processing method, and a program.

Background Art

A technique is disclosed that is related to a trained model which carries out image recognition of an image including a specimen as a subject in pathological diagnosis.

Patent Literature 1 discloses a technique in which unevenness in samples is suppressed by normalizing whole slide image (WSI) data by carrying out pretreatment with respect to each of a plurality of WSIs in which different dyes are used, and the normalized WSI data is applied to a machine learning model.

Citation List

[Patent Literature]

[Patent Literature 1]
Published Japanese Translation of PCT International Application Tokuhyo No. 2021-524630

Summary of Invention

Technical Problem

The technique disclosed in Patent Literature 1 does not consider a relationship between an image for training and an image input into a machine learning model for image recognition in pathological diagnosis. Therefore, the technique disclosed in Patent Literature 1 has a problem that accuracy in image recognition of an image input into a machine learning model may be lowered.

Such a problem causes a false positive in which classification as being malignant is made despite being benign in classification between benignancy and malignancy for classifying a specimen cell as a benign cell or a malignant cell. In a case where a false positive occurs, reinspection is needed even though reinspection is not actually necessary. In a case where a false negative occurs in which classification as being benign is made despite being malignant, there is also a risk of overlooking cancer. Therefore, in the classification between benignancy and malignancy, it is preferable that the accuracy in image recognition of an input image is high.

An example aspect of the present invention is accomplished in view of the above problems, and an example object thereof is to provide a technique for improving accuracy in classification of a specimen cell between benignancy and malignancy in pathological diagnosis.

Solution to Problem

An image processing apparatus in accordance with an example aspect of the present invention includes: a first acquisition means for acquiring a target image which includes a specimen cell as a subject; a second acquisition means for acquiring property information which indicates a property of a training image that has been used to train a prediction model to predict, upon receipt of input of an image including a cell as a subject, whether the cell is a benign cell or a malignant cell; and a generation means for generating an image by changing a property of the target image with reference to the property information.

An image processing method in accordance with an example aspect of the present invention includes: acquiring, by an image processing apparatus, a target image which includes a specimen cell as a subject; acquiring, by the image processing apparatus, property information which indicates a property of a training image that has been used to train a prediction model to predict, upon receipt of input of an image including a cell as a subject, whether the cell is a benign cell or a malignant cell; and generating, by the image processing apparatus, an image by changing a property of the target image with reference to the property information.

A program in accordance with an example aspect of the present invention is a program for causing a computer to function as an image processing apparatus, the program causing the computer to function as: a first acquisition means for acquiring a target image which includes a specimen cell as a subject; a second acquisition means for acquiring property information which indicates a property of a training image that has been used to train a prediction model to predict, upon receipt of input of an image including a cell as a subject, whether the cell is a benign cell or a malignant cell; and a generation means for generating an image by changing a property of the target image with reference to the property information.

Advantageous Effects of Invention

According to an example aspect of the present invention, it is possible to improve accuracy in classification of a specimen cell between benignancy and malignancy in pathological diagnosis.

Brief Description of Drawings

Fig. 1 is a block diagram illustrating a configuration of an image processing apparatus in accordance with a first example embodiment of the present invention.
Fig. 2 is a flowchart illustrating an image processing method in accordance with the first example embodiment of the present invention.
Fig. 3 is a block diagram illustrating a configuration of a classification apparatus in accordance with a second example embodiment of the present invention.
Fig. 4 is a flowchart illustrating an example of a flow of a process in which a generation section in accordance with the second example embodiment of the present invention generates an image by changing a noise level of a target image.
Fig. 5 is a flowchart illustrating an example of a flow of a process in which the generation section in accordance with the second example embodiment of the present invention generates an image by changing a color distribution of a target image.
Fig. 6 is a diagram illustrating an example of a histogram of hue in the second example embodiment of the present invention.
Fig. 7 is a flowchart illustrating an example of a flow of a process in which the generation section in accordance with the second example embodiment of the present invention changes resolution of a target image.
Fig. 8 is a diagram illustrating an example of an image of a micrometer in the second example embodiment of the present invention.
Fig. 9 is a flowchart illustrating another example of a flow of a process in which the generation section in accordance with the second example embodiment of the present invention changes resolution of a target image.
Fig. 10 is a block diagram illustrating an example hardware configuration of the image processing apparatus and the classification apparatus in accordance with the example embodiments of the present invention.

Example Embodiments

[First example embodiment]

The following description will discuss a first example embodiment of the present invention in detail, with reference to the drawings. The present example embodiment is a basic form of example embodiments described later.

(Overview of image processing apparatus 1)

An image processing apparatus 1 in accordance with the present example embodiment is an image processing apparatus that generates an image by changing a property of a target image which includes a specimen cell as a subject. For example, the image processing apparatus 1 generates an image by changing a property of a target image in accordance with a property of a training image that has been used to train a prediction model to predict, upon receipt of input of an image including a cell as a subject, whether the cell is a benign cell or a malignant cell.
A property of an image refers to quality or a feature of an image. Examples of the property include: a noise level indicating a magnitude of noise included in an image; a color distribution constituted by hue, lightness, and chroma in an image; resolution indicating the number of pixels included in a predetermined length in an image; and information indicating a size of a predetermined object included in an image as a subject.
A specific configuration of the prediction model does not limit the present example embodiment, and can be, for example, a convolution neural network (CNN), a recurrent neural network (RNN), or a combination of these networks. Alternatively, a non-neural network type model such as a random forest or a support vector machine can be used.

(Configuration of image processing apparatus 1)

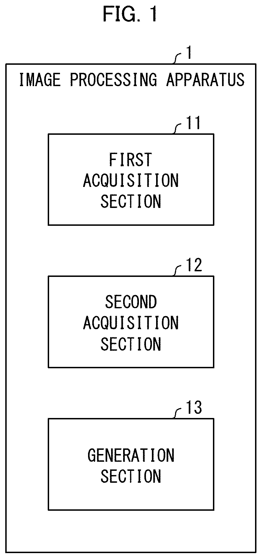

The following description will discuss a configuration of the image processing apparatus 1 in accordance with the present example embodiment, with reference to Fig. 1. Fig. 1 is a block diagram illustrating the configuration of the image processing apparatus 1 in accordance with the present example embodiment.

As illustrated in Fig. 1, the image processing apparatus 1 includes a first acquisition section 11, a second acquisition section 12, and a generation section 13. The first acquisition section 11, the second acquisition section 12, and the generation section 13 are configured to realize the first acquisition means, the second acquisition means, and the generation means in the present example embodiment, respectively.

The first acquisition section 11 acquires a target image which includes a specimen cell as a subject. The first acquisition section 11 supplies the acquired target image to the generation section 13.

The second acquisition section 12 acquires property information indicating a property of a training image that has been used to train a prediction model to predict, upon receipt of input of an image including a cell as a subject, whether the cell is a benign cell or a malignant cell. The second acquisition section 12 supplies the acquired property information to the generation section 13.

The generation section 13 generates an image by changing, with reference to the property information supplied from the second acquisition section 12, a property of the target image supplied from the first acquisition section 11.

As described above, the image processing apparatus 1 in accordance with the present example embodiment employs the configuration of including: the first acquisition section 11 for acquiring a target image which includes a specimen cell as a subject; the second acquisition section 12 for acquiring property information which indicates a property of a training image that has been used to train a prediction model to predict, upon receipt of input of an image including a cell as a subject, whether the cell is a benign cell or a malignant cell; and the generation section 13 for generating an image by changing a property of the target image with reference to the property information.

That is, the image processing apparatus 1 in accordance with the present example embodiment generates an image by changing a property of a target image in accordance with a property of a training image that has been used to train a prediction model. Therefore, according to the image processing apparatus 1 in accordance with the present example embodiment, it is possible to bring about an example advantage of improving accuracy in classification of a specimen cell between benignancy and malignancy in pathological diagnosis.

(Flow of image processing method S1)

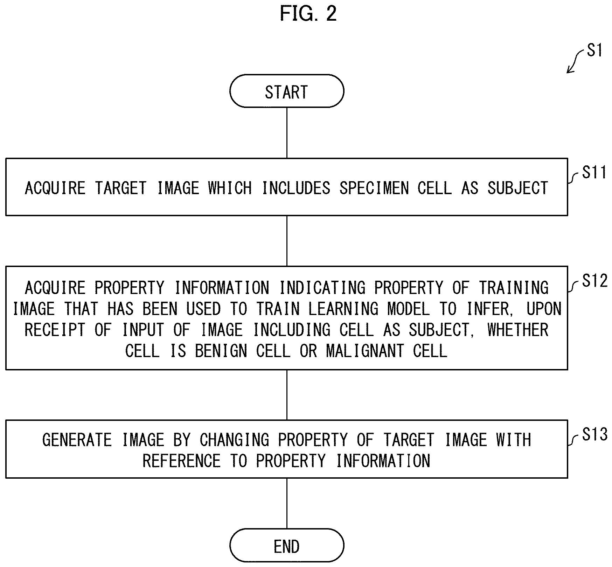

The following description will discuss a flow of an image processing method S1 in accordance with the present example embodiment, with reference to Fig. 2. Fig. 2 is a flowchart illustrating the flow of the image processing method S1 in accordance with the present example embodiment.

(Step S11)

In step S11, the first acquisition section 11 acquires a target image which includes a specimen cell as a subject. The first acquisition section 11 supplies the acquired target image to the generation section 13.

(Step S12)

In step S12, the second acquisition section 12 acquires property information indicating a property of a training image that has been used to train a prediction model to predict, upon receipt of input of an image including a cell as a subject, whether the cell is a benign cell or a malignant cell. The second acquisition section 12 supplies the acquired property information to the generation section 13.

(Step S13)

In step S13, the generation section 13 generates an image by changing, with reference to the property information supplied from the second acquisition section 12, a property of the target image supplied from the first acquisition section 11.

As described above, the image processing method S1 in accordance with the present example embodiment employs the configuration of including: acquiring a target image which includes a specimen cell as a subject; acquiring property information which indicates a property of a training image that has been used to train a prediction model to predict, upon receipt of input of an image including a cell as a subject, whether the cell is a benign cell or a malignant cell; and generating an image by changing a property of the target image with reference to the property information. Therefore, according to the image processing method S1 in accordance with the present example embodiment, an example advantage similar to that of the foregoing image processing apparatus 1 is brought about.

[Second example embodiment]

The following description will discuss a second example embodiment of the present invention in detail, with reference to the drawings. The same reference numerals are given to constituent elements which have functions identical with those described in the first example embodiment, and descriptions as to such constituent elements are omitted as appropriate.

(Overview of classification apparatus 2)

A classification apparatus 2 in accordance with the present example embodiment is an apparatus which classifies a specimen cell as a benign cell or a malignant cell.

For example, the classification apparatus 2 inputs an image including a specimen cell as a subject into a prediction model, which has been trained to predict, upon receipt of input of an image including a cell as a subject, whether the cell is a benign cell or a malignant cell, and thus classifies the specimen cell as a benign cell or a malignant cell.

More specifically, the classification apparatus 2 (i) acquires a target image which includes a specimen cell as a subject and (ii) generates an image while making a property of the target image to conform to a property of a training image that has been used to train a prediction model. Then, the classification apparatus 2 inputs the generated image into the prediction model and classifies the specimen cell as a benign cell or a malignant cell with reference to a result obtained by the prediction model. That is, the classification apparatus 2 includes the configuration of the image processing apparatus 1 described above and inputs an image generated by the image processing apparatus 1 into the prediction model, and thus classifies a specimen cell as a benign cell or a malignant cell.

For example, the training image is an image obtained by digitizing an image which includes a specimen cell as a subject, and the target image is an image obtained by capturing a microscopic image of a specimen cell. In a case where the training image is a digitized image, many training images can be acquired. Therefore, the classification apparatus 2 can heighten accuracy of the prediction model. In a case where the target image is an image obtained by capturing a microscopic image, the classification apparatus 2 can be used, for example, in cytodiagnosis in rapid on-site evaluation (ROSE).

The property of an image and the prediction model are as described above.

(Configuration of classification apparatus 2)

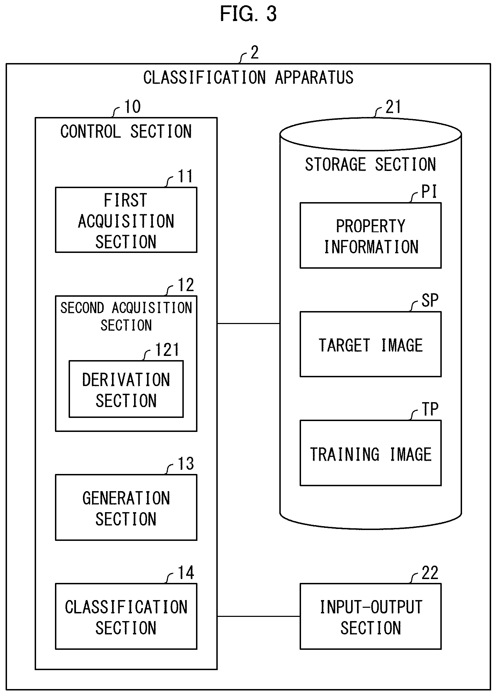

The following description will discuss a configuration of the classification apparatus 2 in accordance with the present example embodiment, with reference to Fig. 3. Fig. 3 is a block diagram illustrating the configuration of the classification apparatus 2 in accordance with the present example embodiment.

As illustrated in Fig. 3, the classification apparatus 2 includes a control section 10, a storage section 21, and an input-output section 22.

In the storage section 21, data referred to by the control section 10 (described later) is stored. Examples of the data stored in the storage section 21 include property information PI indicating a property of a training image TP used to train a prediction model, a target image SP which includes a specimen cell as a subject, and a training image TP.

The input-output section 22 is an interface via which data is acquired from another apparatus connected to the classification apparatus 2 and data is outputted to another apparatus which is connected to the classification apparatus 2. The input-output section 22 supplies data acquired from another apparatus to the control section 10 and outputs data supplied from the control section 10 to another apparatus.

The input-output section 22 may be a communication module that communicates with other apparatuses via a network. A specific configuration of the network does not limit the present example embodiment, and can be, for example, a wireless local area network (LAN), a wired LAN, a wide area network (WAN), a public network, a mobile data communication network, or a combination of these networks.

(Control section 10)

The control section 10 controls constituent elements included in the classification apparatus 2. The control section 10 functions also as a first acquisition section 11, a second acquisition section 12, a generation section 13, and a classification section 14, as illustrated in Fig. 3. The first acquisition section 11, the second acquisition section 12, the generation section 13, and the classification section 14 are configured to realize the first acquisition means, the second acquisition means, the generation means, and the classification means in the present example embodiment, respectively.

The first acquisition section 11 acquires a target image SP which includes a specimen cell as a subject. The first acquisition section 11 causes the storage section 21 to store the acquired target image SP.

The second acquisition section 12 acquires property information PI indicating a property of a training image TP that has been used to train a prediction model to predict, upon receipt of input of an image including a cell as a subject, whether the cell is a benign cell or a malignant cell. The second acquisition section 12 may be configured to acquire the property information PI itself, or may acquire one or more training images TP and derive and acquire property information PI from the one or more training images TP. The second acquisition section 12 causes the storage section 21 to store the acquired property information PI (and training image TP).

Here, examples of the property information PI include property information PI indicating a property which has been obtained by averaging respective properties in a plurality of training images TP and property information PI indicating a property which is a median of variations of respective properties in a plurality of training images TP.

As illustrated in Fig. 3, the second acquisition section 12 includes a derivation section 121.

The derivation section 121 derives property information PI from the training image TP. For example, the derivation section 121 extracts a property of the training image TP and derives property information PI indicating the extracted property. As another example, the derivation section 121 extracts respective properties of a plurality of training images TP, and derives property information PI indicating (i) a property obtained by averaging the extracted properties or (ii) a property which is a median of variations of the respective extracted properties.

The generation section 13 generates an image by changing a property of a target image SP with reference to the property information PI. The generation section 13 supplies the generated image to the classification section 14.

For example, the generation section 13 generates an image by changing a noise level of the target image SP. As another example, the generation section 13 generates an image by changing a color distribution of the target image SP. As yet another example, the generation section 13 generates an image by changing resolution of the target image SP. As still another example, the generation section 13 generates an image by changing a size of an object included in the target image SP.

The generation section 13 may generate an image by changing a plurality of properties of the target image SP. For example, the generation section 13 generates an image by changing a noise level, a color distribution, and resolution of the target image SP.

In a case where the generation section 13 generates an image by changing a plurality of properties of the target image SP, the generation section 13 may generate an image by changing the properties in a predetermined order or may generate an image by changing the plurality of properties of the target image SP regardless of the order.

In an example configuration in which an image is generated by changing properties in a predetermined order, the generation section 13 first generates an image by changing a noise level of a target image SP. Next, the generation section 13 carries out a process of changing a color distribution with respect to the image having the changed noise level, and thus generates an image having the changed color distribution. Then, the generation section 13 carries out a process of changing resolution with respect to the image having the changed color distribution, and thus generates an image having the changed resolution.

A flow of a process which is carried out by the generation section 13 will be described later.

The classification section 14 inputs an image supplied from the generation section 13 into a prediction model and classifies, as a benign cell or a malignant cell, a specimen cell which is included as a subject in the image supplied from the generation section 13. The classification section 14 outputs the classification result via the input-output section 22.

For example, in a case where the prediction result output from the prediction model indicates that the specimen cell is a benign cell, the classification section 14 classifies, as a benign cell, the specimen cell which is included as a subject in the image supplied from the generation section 13. Meanwhile, in a case where the prediction result output from the prediction model indicates that the specimen cell is a malignant cell, the classification section 14 classifies, as a malignant cell, the specimen cell which is included as a subject in the image supplied from the generation section 13.

(Process of generating image by changing noise level)

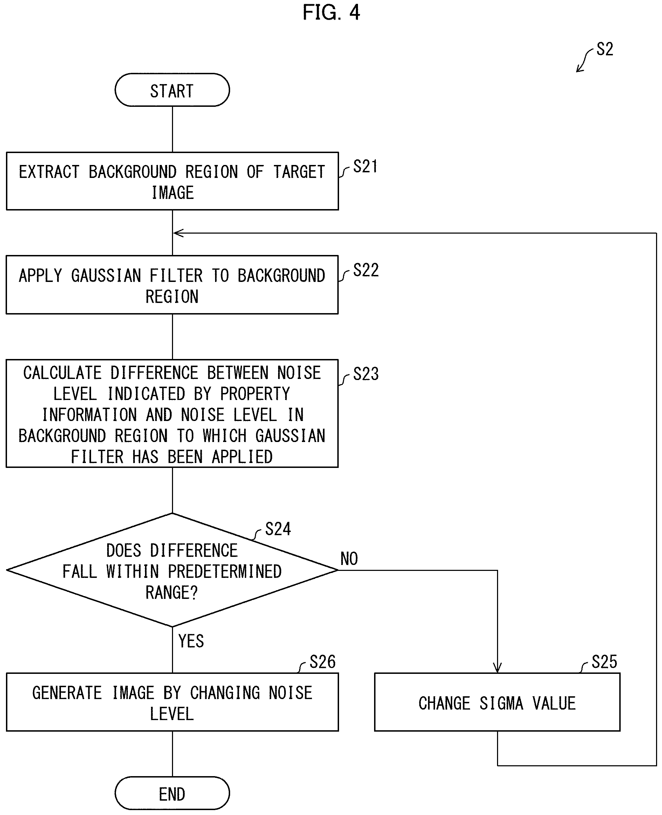

The following description will discuss an example of a process in which the generation section 13 generates an image by changing a noise level of a target image SP, with reference to Fig. 4. Fig. 4 is a flowchart illustrating an example of a flow of a process S2 in which the generation section 13 in accordance with the present example embodiment generates an image by changing a noise level of a target image SP. The following description will discuss a process in which: the property information PI is information indicating a noise level in the training image TP, the noise level being a noise level in a background region that includes no object as a subject; and the generation section 13 calculates a noise level by applying a Gaussian filter.

(Step S21)

In step S21, the generation section 13 extracts, from the target image SP, a background region that is a region in which no object is included as a subject. A method in which the generation section 13 extracts a background region is not particularly limited, and it is possible to employ, for example, a method in which a region having a pixel value that is equal to or less than a predetermined pixel value is extracted as a background region.

(Step S22)

In step S22, the generation section 13 applies a Gaussian filter to the extracted background region. A sigma value of the Gaussian filter to be applied by the generation section 13 is not particularly limited, and can be, for example, a value such as 1.0 or 1.5.

(Step S23)

In step S23, the generation section 13 calculates a noise level in the background region to which the Gaussian filter has been applied. For example, in a case where the property information PI indicates a standard deviation of pixel value as a noise level, the generation section 13 calculates, as a noise level, a standard deviation of pixel value in the background region to which the Gaussian filter has been applied. Then, the generation section 13 calculates a difference between the calculated standard deviation of pixel value in the background region and the standard deviation indicated by the property information PI.

(Step S24)

In step S24, the generation section 13 determines whether or not the calculated difference is within a predetermined range.

(Step S25)

In a case where it has been determined in step S24 that the calculated difference is not within the predetermined range (step S24: NO), in step S25, the generation section 13 changes the sigma value of the Gaussian filter to be applied.
For example, in a case where the standard deviation of pixel value in the background region to which the Gaussian filter has been applied is higher than the standard deviation indicated by the property information PI, the generation section 13 increases the sigma value by 10%. Meanwhile, in a case where the standard deviation of pixel value in the background region to which the Gaussian filter has been applied is lower than the standard deviation indicated by the property information PI, the generation section 13 decreases the sigma value by 10%. That is, the generation section 13 derives a sigma value with which a difference between the noise level indicated by the property information PI and the noise level in the background region of the target image SP falls within a predetermined range.
Then, in order to apply the Gaussian filter having the changed sigma value, the generation section 13 returns to the process of step S22.

(Step S26)

In a case where it has been determined in step S24 that the calculated difference is within the predetermined range (step S24: YES), in step S26, the generation section 13 generates an image by changing the noise level by applying, to the target image SP, the Gaussian filter having the sigma value with which a difference between the noise level indicated by the property information PI and the noise level in the background region of the target image SP falls within a predetermined range. That is, the generation section 13 generates an image by changing the noise level of the target image SP so that the noise level in the extracted background region of the target image SP is brought close to the noise level indicated by the property information PI.
Thus, the generation section 13 generates an image by changing the noise level of the target image SP so that the noise level in the extracted background region of the target image SP is brought close to the noise level indicated by the property information PI. Therefore, the generation section 13 can input an image having a noise level equivalent to the noise level of the training image TP into the prediction model, and this makes it possible to improve accuracy in classification of a specimen cell between benignancy and malignancy in pathological diagnosis.

(Process 1 of generating image by changing color distribution)

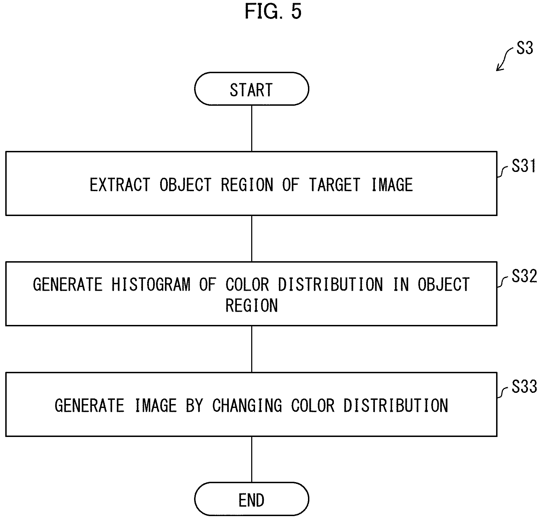

The following description will discuss an example of a process in which the generation section 13 generates an image by changing a color distribution of a target image SP, with reference to Fig. 5. Fig. 5 is a flowchart illustrating an example of a flow of a process S3 in which the generation section 13 in accordance with the present example embodiment generates an image by changing a color distribution of a target image SP. The following description will discuss a case in which the property information PI is information indicating a color distribution in the training image TP, the color distribution being a color distribution in an object region in which an object is included as a subject. For example, the property information PI is information indicating respective histograms of hue, chroma, and lightness in the object region of the training image TP.

(Step S31)

In step S31, the generation section 13 extracts, from the target image SP, an object region that is a region in which an object is included as a subject. A method in which the generation section 13 extracts an object region is not particularly limited, and it is possible to employ, for example, a method in which a region having a pixel value that is equal to or more than a predetermined pixel value is extracted as an object region.

(Step S32)

In step S32, the generation section 13 generates a histogram of a color distribution in the extracted object region as information indicating a color distribution in the extracted object region. For example, the generation section 13 generates respective histograms of hue, chroma, and lightness of the extracted object region.

(Step S33)

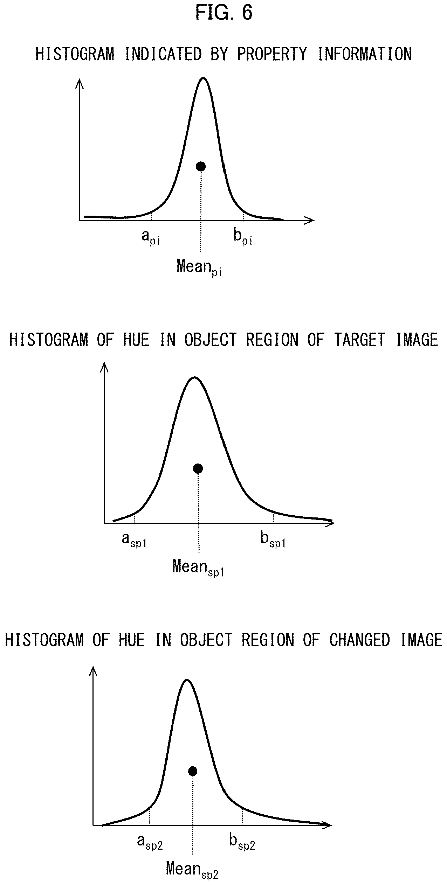

In step S33, the generation section 13 generates an image by changing color distribution in the extracted object region of the target image SP so that the color distribution is brought close to color distribution indicated by the property information PI. The following description will discuss an example of a process which is carried out by the generation section 13 in step S33, with reference to Fig. 6. Fig. 6 is a diagram illustrating an example of a histogram of hue in the present example embodiment. The following description will discuss a histogram of hue, and similar descriptions apply to histograms of chroma and lightness.

In a case where the histogram of hue indicated by the property information PI is a histogram illustrated in the upper part of Fig. 6, the generation section 13 calculates an average value $mean_{pi}$ and a left end $a_{pi}$ and a right end $b_{pi}$ of a 95% confidence interval in the histogram of hue indicated by the property information PI.

Next, in a case where the histogram of hue in the object region of the target image SP generated in step S32 is a histogram illustrated in the middle part of Fig. 6, the generation section 13 similarly calculates an average value $mean_{sp1}$ and a left end $a_{sp1}$ and a right end $b_{sp1}$ of a 95% confidence interval in the histogram of hue in the object region of the target image SP.

Then, the generation section 13 calculates a changed value Out using a formula (1) below.

$$Out = \frac{Var_{poi}}{Var_{tar}}\left(In - mean_{poi}\right) + mean_{tar} \quad \cdot\cdot\cdot \quad （1）$$

Variables in the formula (1) are as follows.

Out: A hue value after change
In: A hue value of a target image SP
$Var_{tar}$: A value obtained by subtracting a lower limit value from an upper limit value of a 95% confidence interval of a hue value In in a target histogram. That is, a value indicated by [right end $b_{pi}$-left end $a_{pi}$].
$Var_{poi1}$: A value obtained by subtracting a lower limit value from an upper limit value of a 95% confidence interval of a hue value In in a histogram of a target image SP. That is, a value indicated by [right end $b_{sp1}$-left end $a_{sp1}$].

That is, the generation section 13 uses the formula (1) to change, in a 95% confidence interval of each histogram, a value $Var_{poi1}$ to conform to a value $Var_{tar}$, which is a counterpart in the target histogram. Here, the value $Var_{poi1}$ is a value obtained by subtracting a lower limit value from an upper limit value of a 95% confidence interval in a certain hue value In in the target image SP. Thus, the certain hue value In is converted into a target hue value.

A histogram of hue in an object region of the image after conversion is illustrated in the lower part of Fig. 6. As illustrated in the lower part of Fig. 6, an average value $mean_{sp2}$ and a left end $a_{sp2}$ and a right end $b_{sp2}$ of a 95% confidence interval in the histogram of hue in the object region after conversion are closer to the average value $mean_{pi}$ and the left end $a_{pi}$ and the right end $b_{pi}$ of the 95% confidence interval in the histogram indicated by the property information PI, as compared with the average value $mean_{sp1}$ and the left end $a_{sp1}$ and the right end $b_{sp1}$ of the 95% confidence interval in the histogram of a color distribution in the object region of the target image SP.

Thus, the generation section 13 generates an image by changing color distribution in the object region of the target image SP so that the color distribution is brought close to the color distribution indicated by the property information PI. Therefore, the generation section 13 can input an image having a color distribution equivalent to the color distribution of the training image TP into the prediction model, and this makes it possible to improve accuracy in classification of a specimen cell between benignancy and malignancy in pathological diagnosis.

(Process 2 of generating image by changing color distribution)

In the process of changing a color distribution of the target image SP, the generation section 13 may change a color distribution of the background region of the target image SP in addition to the object region of the target image SP.

For example, the generation section 13 may change a pixel value RGB of the background region of the target image SP to a predetermined value (e.g., (R,G,B) = (230,230,230) for an image with 256 levels of gray).

As another example, the generation section 13 may be configured to refer to a background region of the training image TP. For example, the generation section 13 may acquire an average value of pixel values of the background region of the training image TP and change pixel values of a background region of a target image SP to the average value. As another example, the generation section 13 extracts respective background regions of a plurality of training images TP and calculates an average value of pixel values of the extracted background regions. Then, the generation section 13 may change pixel values of the background region of the target image SP to the calculated average value. In this case, the generation section 13 may use a median of variations in pixel values, instead of the average value of pixel values.

(Process 1 of generating image by changing resolution)

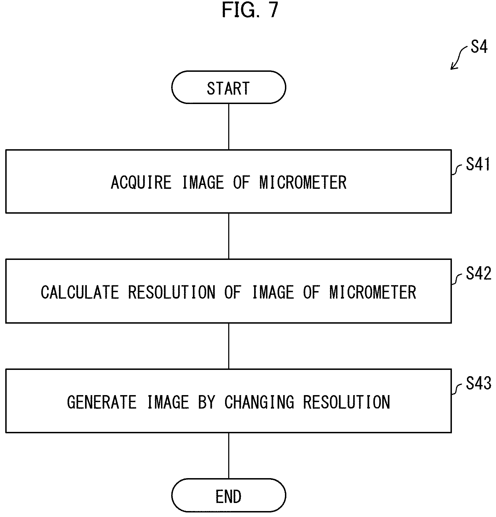

The following description will discuss an example of a process in which the generation section 13 changes resolution of a target image SP, with reference to Fig. 7. Fig. 7 is a flowchart illustrating an example of a flow of a process S4 in which the generation section 13 in accordance with the present example embodiment changes resolution of a target image SP. The following description will discuss a process in which: the property information PI is information indicating resolution of the training image TP; and the generation section 13 refers to an image which includes a micrometer as a subject.

(Step S41)

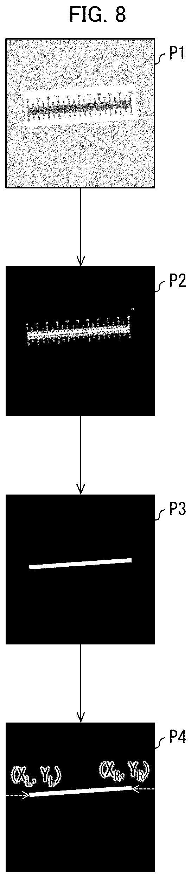

In step S41, the generation section 13 acquires an image of a micrometer captured with the same resolution as the target image SP. The following description will discuss an example of an image of a micrometer, with reference to Fig. 8. Fig. 8 is a diagram illustrating an example of an image of a micrometer in the present example embodiment. As illustrated in an image P1 of Fig. 8, the generation section 13 acquires an image which includes a micrometer as a subject.

(Step S42)

In step S42, the generation section 13 calculates resolution of the image of the micrometer.

For example, the generation section 13 first generates an image by binarizing the image P1, as illustrated in an image P2 of Fig. 8. Next, the generation section 13 carries out a closing process by carrying out a dilation process and an erosion process on the image P2, as illustrated in an image P3 of Fig. 8. Then, the generation section 13 detects coordinates $(X_L, Y_L)$ of a left-end pixel of the micrometer and coordinates $(X_R, Y_R)$ of a right-end pixel of the micrometer as illustrated in an image P4 of Fig. 8 (where a direction along the horizontal direction of the image P4 is defined as an X-axis, and a direction perpendicular to the X-axis is defined as a Y-axis), and calculates resolution Resolution using the following formula (2).

$$Resolution = L/(\sqrt{(X_R - X_L)^2 + (Y_R - Y_L)^2} \quad \cdots \quad (2)$$

In the formula (2), L represents a length of the micrometer.

That is, the generation section 13 calculates resolution by calculating, using the formula (2), the number of pixels of the target image SP included in a predetermined length.

(Step S43)

In step S43, the generation section 13 generates an image by changing the calculated resolution to resolution indicated by the property information PI.

Thus, the generation section 13 generates an image by changing resolution of the target image SP so that the resolution indicated by the property information PI is equal to the resolution of the target image SP. Therefore, it is possible to input, into the prediction model, an image in which a size of an object included in the training image TP as a subject is equivalent to a size of an object included in the target image SP as a subject, and this makes it possible to improve accuracy in classification of a specimen cell between benignancy and malignancy in pathological diagnosis.

(Process 2 of generating image by changing resolution)

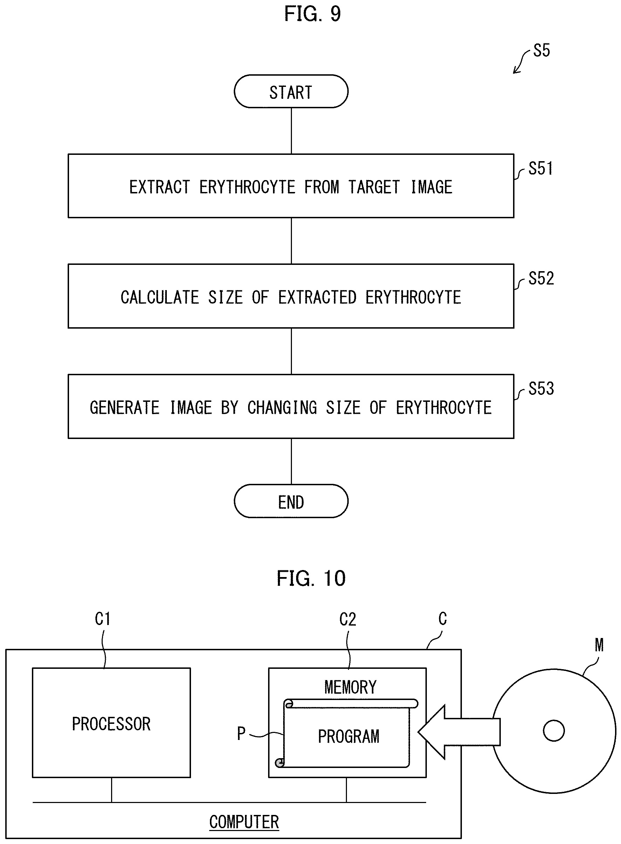

The following description will discuss another example of a process in which the generation section 13 changes resolution of a target image SP, with reference to Fig. 9. Fig. 9 is a flowchart illustrating another example of a flow of a process S5 in which the generation section 13 in accordance with the present example embodiment changes resolution of a target image SP. The following description will discuss a configuration in which: the property information PI is information indicating a size of a predetermined object which is included in the training image TP as a subject; and the generation section 13 extracts a predetermined object which is included as a subject in the target image SP.

Here, the predetermined object is not particularly limited, and is preferably an object with a small individual difference in size. Examples of the predetermined object include an erythrocyte and a lymphocyte. In this configuration, the generation section 13 uses an erythrocyte or a lymphocyte with a small individual difference as a reference for a size of an object included in the target image SP. Therefore, it is possible to suitably calculate a size of an object. The following description will discuss a case in which the predetermined object is an erythrocyte.

(Step S51)

In step S51, the generation section 13 extracts an erythrocyte included as a subject in the target image SP as a predetermined object included as a subject in the target image SP. Examples of a method in which the generation section 13 extracts an erythrocyte which is included in the target image SP as a subject include a method using known deep learning segmentation (DL segmentation).

(Step S52)

In step S52, the generation section 13 calculates a size of the extracted erythrocyte. The generation section 13 may elliptically approximate the extracted erythrocyte and calculate a major axis and a minor axis.

In a case where a plurality of erythrocytes are extracted, for example, the generation section 13 may calculate a major axis and a minor axis of an erythrocyte having the largest area. As another example, the generation section 13 may calculate a major axis and a minor axis of an erythrocyte which has a median of variations in areas of the plurality of erythrocytes. As yet another example, the generation section 13 may calculate an average major axis and an average minor axis of the plurality of erythrocytes.

(Step S53)

In step S53, the generation section 13 generates an image by changing resolution of the target image SP so that the calculated size (major axis and minor axis) of the erythrocyte is a size (major axis and minor axis) indicated by the property information PI.

Thus, the generation section 13 generates an image by changing resolution of the target image SP so that a size of a predetermined object indicated by the property information PI is equivalent to a size of a predetermined object included in the target image SP as a subject. Therefore, it is possible to input, into the prediction model, an image in which a size of an object included in the training image TP as a subject is equivalent to a size of an object included in the target image SP as a subject, and this makes it possible to improve accuracy in classification of a specimen cell between benignancy and malignancy in pathological diagnosis.

(Example advantage of classification apparatus 2)

As described above, the classification apparatus 2 in accordance with the present example embodiment generates an image by changing a property of a target image SP which includes a specimen cell as a subject, with reference to property information PI which indicates a property of a training image TP that has been used to train a prediction model to predict, upon receipt of input of an image including a cell as a subject, whether the cell is a benign cell or a malignant cell. Furthermore, the classification apparatus 2 in accordance with the present example embodiment employs the configuration in which the generated image is input into the prediction model.

That is, the classification apparatus 2 in accordance with the present example embodiment generates an image by changing a property of a target image SP in accordance with a property of a training image TP that has been used to train a prediction model, and then the image is input into the prediction model. Therefore, according to the classification apparatus 2 in accordance with the present example embodiment, it is possible to bring about an example advantage of improving accuracy in classification of a specimen cell between benignancy and malignancy in pathological diagnosis.

[Software implementation example]

Some or all of the functions of each of the image processing apparatus 1 and the classification apparatus 2 may be implemented by hardware such as an integrated circuit (IC chip), or may be implemented by software.

In the latter case, the image processing apparatus 1 and the classification apparatus 2 are implemented by, for example, a computer that executes instructions of a program that is software implementing the foregoing functions. Fig. 10 illustrates an example of such a computer (hereinafter, referred to as "computer C"). The computer C includes at least one processor C1 and at least one memory C2. The memory C2 stores a program P for causing the computer C to operate as the image processing apparatus 1 and the classification apparatus 2. The processor C1 of the computer C retrieves the program P from the memory C2 and executes the program P, so that the functions of the image processing apparatus 1 and the classification apparatus 2 are implemented.

As the processor C1, for example, it is possible to use a central processing unit (CPU), a graphic processing unit (GPU), a digital signal processor (DSP), a micro processing unit (MPU), a floating point number processing unit (FPU), a physics processing unit (PPU), a microcontroller, or a combination of these. Examples of the memory C2 include a flash memory, a hard disk drive (HDD), a solid state drive (SSD), and a combination thereof.

Note that the computer C can further include a random access memory (RAM) in which the program P is loaded in a case where the program P is executed and in which various kinds of data are temporarily stored. The computer C can further include a communication interface for carrying out transmission and reception of data with other apparatuses. The computer C can further include an input-output interface for connecting input-output apparatuses such as a keyboard, a mouse, a display and a printer.

The program P can be stored in a computer C-readable, non-transitory, and tangible storage medium M. The storage medium M can be, for example, a tape, a disk, a card, a semiconductor memory, a programmable logic circuit, or the like. The computer C can obtain the program P via the storage medium M. The program P can be transmitted via a transmission medium. The transmission medium can be, for example, a communication network, a broadcast wave, or the like. The computer C can obtain the program P also via such a transmission medium.

[Additional remark 1]

The present invention is not limited to the foregoing example embodiments, but may be altered in various ways by a skilled person within the scope of the claims. For example, the present invention also encompasses, in its technical scope, any example embodiment derived by appropriately combining technical means disclosed in the foregoing example embodiments.

[Additional remark 2]

Some or all of the foregoing example embodiments can also be described as below. Note, however, that the present invention is not limited to the following supplementary notes.

(Supplementary note 1)

An image processing apparatus, including: a first acquisition means for acquiring a target image which includes a specimen cell as a subject; a second acquisition means for acquiring property information which indicates a property of a training image that has been used to train a prediction model to predict, upon receipt of input of an image including a cell as a subject, whether the cell is a benign cell or a malignant cell; and a generation means for generating an image by changing a property of the target image with reference to the property information.

(Supplementary note 2)

The image processing apparatus according to supplementary note 1, in which: the property information is information indicating a noise level in the training image, the noise level being a noise level in a background region that includes no object as a subject; and the generation means extracts a background region from the target image and generates an image by changing a noise level in the target image so that a noise level in the background region of the target image which has been extracted is brought close to the noise level indicated by the property information.

(Supplementary note 3)

The image processing apparatus according to supplementary note 2, in which: the generation means calculates a noise level by applying a Gaussian filter to the background region of the target image; and the generation means derives a sigma value with which a difference between the noise level indicated by the property information and the noise level in the background region of the target image falls within a predetermined range.

(Supplementary note 4)

The image processing apparatus according to any one of supplementary notes 1 through 3, in which: the property information is information indicating a color distribution in the training image, the color distribution being a color distribution in an object region that includes an object as a subject; and the generation means extracts an object region from the target image and generates an image by changing a color distribution in the object region of the target image which has been extracted is brought close to the color distribution indicated by the property information.

(Supplementary note 5)

The image processing apparatus according to supplementary note 4, in which: the property information is information indicating respective histograms of hue, chroma, and lightness in the object region of the training image.

(Supplementary note 6)

The image processing apparatus according to any one of supplementary notes 1 through 5, in which: the property information is information indicating resolution of the training image; and the generation means generates an image by changing resolution of the target image to the resolution indicated by the property information with reference to an image of a micrometer captured in resolution that is identical with that of the target image.

(Supplementary note 7)

The image processing apparatus according to any one of supplementary notes 1 through 6, in which: the property information is information indicating a size of a predetermined object included in the training image as a subject; and the generation means extracts a predetermined object which is included in the target image as a subject and generates an image by changing resolution of the target image so that a size of the predetermined object in the target image which has been extracted is the size indicated by the property information.

(Supplementary note 8)

The image processing apparatus according to supplementary note 7, in which: the predetermined object is an erythrocyte or a lymphocyte.

(Supplementary note 9)

The image processing apparatus according to any one of supplementary notes 1 through 8, in which: the second acquisition means acquires the training image; and the second acquisition means includes a derivation means for deriving property information from the training image.

(Supplementary note 10)

The image processing apparatus according to any one of supplementary notes 1 through 9, in which: the second acquisition means acquires (i) property information indicating a property which has been obtained by averaging respective properties in a plurality of training images or (ii) property information indicating a property which is a median of variations of respective properties in a plurality of training images.

(Supplementary note 11)

The image processing apparatus according to any one of supplementary notes 1 through 10, in which: the training image is an image obtained by digitizing an image which includes a specimen cell as a subject; and the target image is an image obtained by capturing a microscopic image of a specimen cell.

(Supplementary note 12)

A classification apparatus: including a classification means for inputting an image generated by an image processing apparatus according to supplementary note 1 into the prediction model, and classifying, as a benign cell or a malignant cell, a specimen cell which is included as a subject in the image generated by the image processing apparatus.

(Supplementary note 13)

An image processing method, including: acquiring, by an image processing apparatus, a target image which includes a specimen cell as a subject; acquiring, by the image processing apparatus, property information which indicates a property of a training image that has been used to train a prediction model to predict, upon receipt of input of an image including a cell as a subject, whether the cell is a benign cell or a malignant cell; and generating, by the image processing apparatus, an image by changing a property of the target image with reference to the property information.

(Supplementary note 14)

A program for causing a computer to function as an image processing apparatus, the program causing the computer to function as: a first acquisition means for acquiring a target image which includes a specimen cell as a subject; a second acquisition means for acquiring property information which indicates a property of a training image that has been used to train a prediction model to predict, upon receipt of input of an image including a cell as a subject, whether the cell is a benign cell or a malignant cell; and a generation means for generating an image by changing a property of the target image with reference to the property information.

[Additional remark 3]

Some or all of the foregoing example embodiments can also be described as below.
An image processing apparatus, including at least one processor, the at least one processor carrying out: a first acquisition process of acquiring a target image which includes a specimen cell as a subject; a second acquisition process of acquiring property information which indicates a property of a training image that has been used to train a prediction model to predict, upon receipt of input of an image including a cell as a subject, whether the cell is a benign cell or a malignant cell; and a generation process of generating an image by changing a property of the target image with reference to the property information.
Note that the image processing apparatus can further include a memory. The memory can store a program for causing the at least one processor to carry out the first acquisition process, the second acquisition process, and the generation process. The program can be stored in a computer-readable non-transitory tangible storage medium.

Reference Signs List

1:      Image processing apparatus
2:      Classification apparatus
10:     Control section
11:     First acquisition section
12:     Second acquisition section
13:     Generation section
14:     Classification section
121:    Derivation section

**Claims**

1. An image processing apparatus, comprising:

     a first acquisition means for acquiring a target image which includes a specimen cell as a subject;
     a second acquisition means for acquiring property information which indicates a property of a training image that has been used to train a prediction model to predict, upon receipt of input of an image including a cell as a subject, whether the cell is a benign cell or a malignant cell; and

a generation means for generating an image by changing a property of the target image with reference to the property information.

2. The image processing apparatus according to claim 1, wherein:

the property information is information indicating a noise level in the training image, the noise level being a noise level in a background region that includes no object as a subject; and

the generation means extracts a background region from the target image and generates an image by changing a noise level in the target image so that a noise level in the background region of the target image which has been extracted is brought close to the noise level indicated by the property information.

3. The image processing apparatus according to claim 2, wherein:

the generation means calculates a noise level by applying a Gaussian filter to the background region of the target image; and

the generation means derives a sigma value with which a difference between the noise level indicated by the property information and the noise level in the background region of the target image falls within a predetermined range.

4. The image processing apparatus according to any one of claims 1 through 3, wherein:

the property information is information indicating a color distribution in the training image, the color distribution being a color distribution in an object region that includes an object as a subject; and

the generation means extracts an object region from the target image and generates an image by changing a color distribution in the object region of the target image which has been extracted is brought close to the color distribution indicated by the property information.

5. The image processing apparatus according to claim 4, wherein:
the property information is information indicating respective histograms of hue, chroma, and lightness in the object region of the training image.

6. The image processing apparatus according to any one of claims 1 through 5, wherein:

the property information is information indicating resolution of the training image; and

the generation means generates an image by changing resolution of the target image to the resolution indicated by the property information with reference to an image of a micrometer captured in resolution that is identical with that of the target image.

7. The image processing apparatus according to any one of claims 1 through 6, wherein:

the property information is information indicating a size of a predetermined object included in the training image as a subject; and

the generation means extracts a predetermined object which is included in the target image as a subject and generates an image by changing resolution of the target image so that a size of the predetermined object in the target image which has been extracted is the size indicated by the property information.

8. The image processing apparatus according to claim 7, wherein:
the predetermined object is an erythrocyte or a lymphocyte.

9. The image processing apparatus according to any one of claims 1 through 8, wherein:

the second acquisition means acquires the training image; and

the second acquisition means includes a derivation means for deriving property information from the training image.

10. The image processing apparatus according to any one of claims 1 through 9, wherein:

the second acquisition means acquires (i) property information indicating a property which has been obtained by averaging respective properties in a plurality of training images or (ii) property information indicating a property which is a median of variations of respective properties in a plurality of training images.

11. The image processing apparatus according to any one of claims 1 through 10, wherein:

the training image is an image obtained by digitizing an image which includes a specimen cell as a subject; and
the target image is an image obtained by capturing a microscopic image of a specimen cell.

12. A classification apparatus, comprising:
a classification means for inputting an image generated by an image processing apparatus according to claim 1 into the prediction model, and classifying, as a benign cell or a malignant cell, a specimen cell which is included as a subject in the image generated by the image processing apparatus.

13. An image processing method, comprising:

acquiring, by an image processing apparatus, a target image which includes a specimen cell as a subject;
acquiring, by the image processing apparatus, property information which indicates a property of a training image that has been used to train a prediction model to predict, upon receipt of input of an image including a cell as a subject, whether the cell is a benign cell or a malignant cell; and
generating, by the image processing apparatus, an image by changing a property of the target image with reference to the property information.

14. A program for causing a computer to function as an image processing apparatus, said program causing the computer to function as:

a first acquisition means for acquiring a target image which includes a specimen cell as a subject;
a second acquisition means for acquiring property information which indicates a property of a training image that has been used to train a prediction model to predict, upon receipt of input of an image including a cell as a subject, whether the cell is a benign cell or a malignant cell; and
a generation means for generating an image by changing a property of the target image with reference to the property information.

FIG. 1

1

IMAGE PROCESSING APPARATUS

11

FIRST
ACQUISITION
SECTION

12

SECOND
ACQUISITION
SECTION

13

GENERATION
SECTION

FIG. 2

S1

START

ACQUIRE TARGET IMAGE WHICH INCLUDES SPECIMEN CELL AS SUBJECT    S11

ACQUIRE PROPERTY INFORMATION INDICATING PROPERTY OF TRAINING    S12
IMAGE THAT HAS BEEN USED TO TRAIN LEARNING MODEL TO INFER, UPON
RECEIPT OF INPUT OF IMAGE INCLUDING CELL AS SUBJECT, WHETHER
CELL IS BENIGN CELL OR MALIGNANT CELL

GENERATE IMAGE BY CHANGING PROPERTY OF TARGET IMAGE WITH    S13
REFERENCE TO PROPERTY INFORMATION

END

FIG. 3

CLASSIFICATION APPARATUS 2

CONTROL SECTION 10

FIRST ACQUISITION SECTION 11

SECOND ACQUISITION SECTION 12

DERIVATION SECTION 121

GENERATION SECTION 13

CLASSIFICATION SECTION 14

STORAGE SECTION 21

PROPERTY INFORMATION PI

TARGET IMAGE SP

TRAINING IMAGE TP

INPUT-OUTPUT SECTION 22

FIG. 4

S2

```
┌─────────────┐
│    START    │
└─────────────┘
       │
       ▼
┌──────────────────────────────┐
│ EXTRACT BACKGROUND REGION OF │  S21
│         TARGET IMAGE         │
└──────────────────────────────┘
       │
       ▼ ◄──────────────────────────────────────┐
┌──────────────────────────────┐                │
│ APPLY GAUSSIAN FILTER TO      │  S22           │
│       BACKGROUND REGION       │                │
└──────────────────────────────┘                │
       │                                         │
       ▼                                         │
┌──────────────────────────────┐                │
│ CALCULATE DIFFERENCE BETWEEN  │  S23           │
│   NOISE LEVEL INDICATED BY    │                │
│  PROPERTY INFORMATION AND     │                │
│   NOISE LEVEL IN BACKGROUND   │                │
│  REGION TO WHICH GAUSSIAN     │                │
│    FILTER HAS BEEN APPLIED    │                │
└──────────────────────────────┘                │
       │                                         │
       ▼                                         │
      ╱╲                                         │
     ╱  ╲  S24                                   │
    ╱ DOES╲                                      │
   ╱DIFFERENCE╲  NO                 ┌────────────────────┐
  ╱ FALL WITHIN ╲───────────►      │  CHANGE SIGMA VALUE │ S25
   ╲PREDETERMINED╱                  └────────────────────┘
    ╲  RANGE?  ╱
     ╲       ╱
      ╲    ╱
       │ YES
       ▼
┌──────────────────────────────┐
│ GENERATE IMAGE BY CHANGING    │  S26
│         NOISE LEVEL           │
└──────────────────────────────┘
       │
       ▼
┌─────────────┐
│     END     │
└─────────────┘
```

FIG. 5

S3

START

EXTRACT OBJECT REGION OF TARGET IMAGE — S31

GENERATE HISTOGRAM OF COLOR DISTRIBUTION IN OBJECT REGION — S32

GENERATE IMAGE BY CHANGING COLOR DISTRIBUTION — S33

END

# FIG. 6

HISTOGRAM INDICATED BY PROPERTY INFORMATION

$a_{pi}$  $b_{pi}$

$\mathrm{Mean}_{pi}$

HISTOGRAM OF HUE IN OBJECT REGION OF TARGET IMAGE

$a_{sp1}$  $b_{sp1}$

$\mathrm{Mean}_{sp1}$

HISTOGRAM OF HUE IN OBJECT REGION OF CHANGED IMAGE

$a_{sp2}$  $b_{sp2}$

$\mathrm{Mean}_{sp2}$

FIG. 7

S4

START

ACQUIRE IMAGE OF MICROMETER S41

CALCULATE RESOLUTION OF IMAGE OF MICROMETER S42

GENERATE IMAGE BY CHANGING RESOLUTION S43

END

FIG. 8

FIG. 9

S5

START

EXTRACT ERYTHROCYTE FROM TARGET IMAGE — S51

CALCULATE SIZE OF EXTRACTED ERYTHROCYTE — S52

GENERATE IMAGE BY CHANGING SIZE OF ERYTHROCYTE — S53

END

FIG. 10

C1          C2    C        M

MEMORY

PROCESSOR

P — PROGRAM

COMPUTER

## INTERNATIONAL SEARCH REPORT

| International application No. |
|---|
| **PCT/JP2022/021048** |

### A. CLASSIFICATION OF SUBJECT MATTER

*G01N 33/48*(2006.01)i
FI: G01N33/48 M

According to International Patent Classification (IPC) or to both national classification and IPC

### B. FIELDS SEARCHED

Minimum documentation searched (classification system followed by classification symbols)

G01N33/48; A61B5/00; A61B6/00; A61B10/00; G06N3/00-3/12; G06N7/08-99/00; G06T7/00; G16H30/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2019/159821 A1 (NIIGATA UNIVERSITY) 22 August 2019 (2019-08-22) claims, paragraphs [0022]-[0026] | 1-14 |
| Y | | 1-14 |
| Y | WO 2022/064763 A1 (FUJIFILM CORP.) 31 March 2022 (2022-03-31) paragraphs [0013]-[0015], [0017], [0066]-[0070], [0072]-[0075] | 1-14 |
| A | JP 2021-506022 A (VENTANA MEDICAL SYSTEMS, INC.) 18 February 2021 (2021-02-18) paragraph [0130] | 1-14 |
| A | JP 2020-518915 A (PASCHALAKIS, Stavros) 25 June 2020 (2020-06-25) abstract | 1-14 |
| A | CN 112435743 A (SHANGHAI GENNRAL HOSPITAL) 02 March 2021 (2021-03-02) abstract, claims | 1-14 |
| A | JP 2018-535471 A (KONINKLIJKE PHILIPS N. V.) 29 November 2018 (2018-11-29) abstract | 1-14 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 July 2022** | **26 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2022/021048** |

### C. DOCUMENTS CONSIDERED TO BE RELEVANT

| Category* | Citation of document, with indication, where appropriate, of the relevant passages | Relevant to claim No. |
|---|---|---|
| A | JP 2019-36255 A (WHAT'S KK) 07 March 2019 (2019-03-07)<br>abstract, claims 1, 12 | 1-14 |
| A | CN 112348059 A (BEIHANG UNIVERSITY) 09 February 2021 (2021-02-09)<br>claims | 1-14 |
| A | CN 112215801 A (BEIHANG UNIVERSITY) 12 January 2021 (2021-01-12)<br>paragraph [0032] | 1-14 |
| A | CN 111564205 A (HUNAN PINXIN BIOENGINEERING CO., LTD.) 21 August 2020<br>(2020-08-21)<br>abstract, paragraph [0075] | 1-14 |
| A | US 2020/0167910 A1 (INTERNATIONAL BUSINESS MACHINES CORP.) 28 May 2020<br>(2020-05-28)<br>abstract, claims | 1-14 |
| A | WO 2022/070491 A1 (SHIMADZU CORP.) 07 April 2022 (2022-04-07)<br>entire text, all drawings | 1-14 |
| A | CN 108172278 A (BEIHANG UNIVERSITY) 15 June 2018 (2018-06-15)<br>abstract, claims | 1-14 |
| A | WO 2019/102829 A1 (OSAKA UNIVERSITY) 31 May 2019 (2019-05-31)<br>abstract, claims | 1-14 |
| A | JP 2021-515240 A (GOOGLE LLC) 17 June 2021 (2021-06-17)<br>abstract, claims | 1-14 |
| A | JP 2021-532350 A (THE REGENTS OF THE UNIVERSITY OF COLORADO A BODY<br>CORPORATE) 25 November 2021 (2021-11-25)<br>abstract, claims | 1-14 |
| A | JP 2020-107239 A (CYGAMES CO., LTD.) 09 July 2020 (2020-07-09)<br>entire text, all drawings | 1-14 |
| A | JP 2019-139370 A (PANASONIC INTELLECTUAL PROPERTY MANAGEMENT CO.,<br>LTD.) 22 August 2019 (2019-08-22)<br>entire text, all drawings | 1-14 |

Form PCT/ISA/210 (second sheet) (January 2015)

# EP 4 530 627 A1

## INTERNATIONAL SEARCH REPORT
### Information on patent family members

International application No.

**PCT/JP2022/021048**

| Patent document cited in search report | | | Publication date (day/month/year) | Patent family member(s) | | | Publication date (day/month/year) |
|---|---|---|---|---|---|---|---|
| WO | 2019/159821 | A1 | 22 August 2019 | EP 3754334 A1 paragraphs [0035]-[0039], claims<br>US 2021/0035300 A1<br>CN 111727371 A<br>KR 10-2020-0121318 A | | | |
| WO | 2022/064763 | A1 | 31 March 2022 | (Family: none) | | | |
| JP | 2021-506022 | A | 18 February 2021 | WO 2019/110583 A1 paragraph [0145]<br>US 2020/0342597 A1<br>EP 3721373 A1<br>CN 111417958 A | | | |
| JP | 2020-518915 | A | 25 June 2020 | WO 2018/200840 A1 abstract<br>EP 3616120 A1<br>CN 111095261 A | | | |
| CN | 112435743 | A | 02 March 2021 | (Family: none) | | | |
| JP | 2018-535471 | A | 29 November 2018 | WO 2017/051187 A1 abstract<br>US 2018/0357765 A1<br>EP 3353740 A1<br>CN 108140236 A | | | |
| JP | 2019-36255 | A | 07 March 2019 | (Family: none) | | | |
| CN | 112348059 | A | 09 February 2021 | (Family: none) | | | |
| CN | 112215801 | A | 12 January 2021 | (Family: none) | | | |
| CN | 111564205 | A | 21 August 2020 | (Family: none) | | | |
| US | 2020/0167910 | A1 | 28 May 2020 | US 2021/0097686 A1 | | | |
| WO | 2022/070491 | A1 | 07 April 2022 | (Family: none) | | | |
| CN | 108172278 | A | 15 June 2018 | (Family: none) | | | |
| WO | 2019/102829 | A1 | 31 May 2019 | (Family: none) | | | |
| JP | 2021-515240 | A | 17 June 2021 | WO 2019/199392 A1 abstract, claims<br>US 2021/0018742 A1<br>EP 3776458 A1 | | | |
| JP | 2021-532350 | A | 25 November 2021 | WO 2020/028313 A1 abstract, claims<br>US 2021/0303818 A1<br>EP 3814749 A1<br>CN 113330292 A | | | |
| JP | 2020-107239 | A | 09 July 2020 | US 2021/0327104 A1 abstract, claims<br>WO 2020/138182 A1<br>CN 113228111 A | | | |
| JP | 2019-139370 | A | 22 August 2019 | (Family: none) | | | |

Form PCT/ISA/210 (patent family annex) (January 2015)

**EP 4 530 627 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021524630 W